# EUROPEAN PATENT APPLICATION

(11) **EP 4 243 151 A1**
(43) Date of publication of application: **13.09.2023**
(21) Application number: 22899605.4
(22) Date of filing: 05.01.2022
(51) Int. Cl.: H01M 10/0567, H01M 10/0569, H01M 10/052, H01M 10/0525

(54) **ELECTROLYTE, SECONDARY BATTERY USING SAME, BATTERY MODULE, BATTERY PACK, AND ELECTRICAL DEVICE**

(71) Applicant: Contemporary Amperex Technology Co., Limited, Ningde City, Fujian 352100 (CN)
(72) Inventor: SHA, Ying, Ningde City, Fujian 352100 (CN); XIE, Lan, Ningde City, Fujian 352100 (CN); LIN, Zhen, Ningde City, Fujian 352100 (CN)
(74) Representative: Gong, Jinping
(86) International application number: PCT/CN2022/070280
(87) International publication number: WO 2023/130249

(57) **Abstract**

The present application provides an electrolyte for preventing erosion of a positive electrode material and dissolution of a transition metal, and a secondary battery using the electrolyte. The electrolyte comprises an organic solvent and one or more of a compound (A) represented by the above general formula (I) or general formula (II), wherein the content of the compound (A) in the electrolyte is 0.01 wt%-5 wt% and the content of ethylene carbonate in the organic solvent is less than or equal to 3 wt%. The secondary battery of the present invention has an excellent cycling life and storage performance under high voltage operating conditions.

## Description

### Technical Field

The present application relates to the technical field of secondary batteries, and in particular to an electrolyte for a secondary battery and a secondary battery using same, and a battery module, a battery pack, and a power consuming device.

### Background Art

In recent years, with the increasing application range, secondary batteries are widely used in energy storage power systems such as hydraulic power, thermal power, wind power and solar power stations, as well as many fields such as electric tools, electric bicycles, electric motorcycles, electric vehicles, military equipment, and aerospace. The secondary batteries generally consist of a positive electrode, a negative electrode, an electrolyte, etc. For example, in lithium-ion secondary batteries, mutual conversion between chemical energy and electrical energy is generally achieved by intercalation and deintercalation of lithium ions in positive and negative electrodes and transfer of charges through an electrolyte in an electrolyte solution.

However, in the prior art, there are always problems such as collapse of a structure of a positive electrode material and dissolution of transition metals therein due to corrosion of the positive electrode material by a solvent, etc., contained in the electrolyte, and thus resulting in cell capacity attenuation, cycling life reduction, etc., of the secondary batteries. Particularly, in high-voltage system secondary batteries, since the surface of the positive electrode material is corroded, when the secondary batteries are charged to a high voltage, an oxidizing power of a positive electrode active material becomes strong, the surface of the positive electrode will be continuously oxidized, and thus the cell capacity attenuation of the secondary batteries is accelerated.

### Summary of the Invention

In view of the above-mentioned technical problems, the present application is made with an aim to provide an electrolyte for a secondary battery to prevent erosion of a positive electrode material and dissolution of a transition metal, and a secondary battery using the electrolyte, thus improving the cycling life and the storage performance of the secondary battery, particularly the secondary battery operating at a high voltage (≥ 4.35 V).

A first aspect of the present application provides an electrolyte, wherein the electrolyte comprises an organic solvent and one or more of a compound (A) represented by the following general formula (I) or general formula (II), 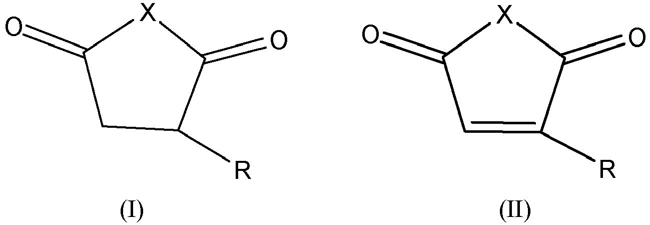
in the general formula (I) or general formula (II),
X is any one element selected from O, S, and N,
when X is O, R comprises one or more of a cyano or isocyanate group, a trimethylsilyl, a straight-chain alkyl or branched-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; when X is S or N, R comprises one or more of a hydrogen atom, a cyano or isocyanate group, a trimethylsilyl, a straight-chain alkyl or branched-chain alkyl with 1-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen;
a content of the compound (A) in the electrolyte is 0.01 wt%-5 wt%; and
a content of ethylene carbonate in the organic solvent is less than or equal to 3 wt%.

Therefore, the compound (A) can capture transition metal ions dissolved in the electrolyte and can form a film on a positive electrode to protect the positive electrode material. By controlling the content of the ethylene carbonate, a penetration corrosion of the solvent on the positive electrode material and dissolution of transition metals in the positive electrode material are reduced, and thus the cycling life and the storage performance of the secondary battery are improved, particularly the secondary battery can still maintain the cycling life and the storage performance under a high voltage (≥ 4.35 V).

In any embodiment, in the general formula (I) or general formula (II), when X is O, R comprises one or more of a cyano or isocyanate group, a trimethylsilyl, a straight-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; and when X is S or N, R comprises one or more of a cyano or isocyanate group, a trimethylsilyl, a hydrogen atom, a straight-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen.

Optionally, when X is O, R comprises one or more of a cyano or isocyanate group, a trimethylsilyl, a straight-chain alkyl with 2-3 carbon atoms, a straight-chain alkyl with 1-3 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; and when X is S or N, R comprises one or more of a cyano or isocyanate group, a trimethylsilyl, a hydrogen atom, a straight-chain alkyl with 2-3 carbon atoms, a straight-chain alkyl with 1-3 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen.

Therefore, a protective effect of the compound (A) on the positive electrode material and the cycling life and storage performance of the secondary battery can be further improved.

In any embodiment, the compound (A) is one or more selected from the following compounds (A1)-(A12):

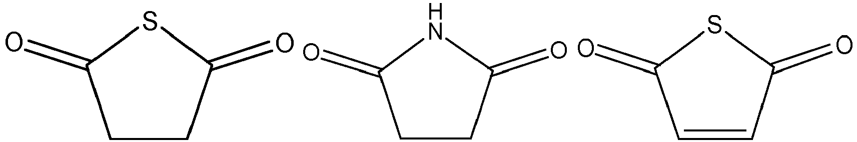

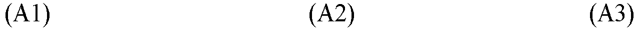

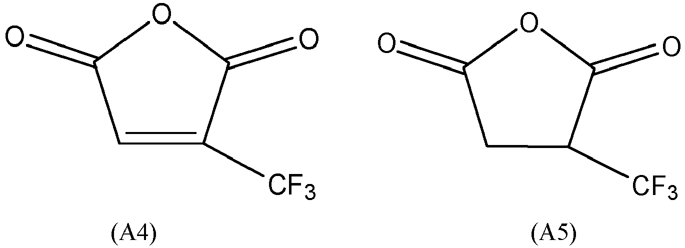

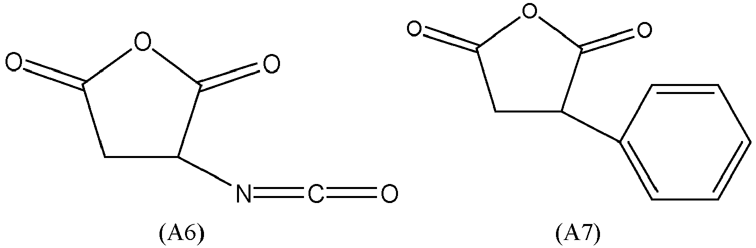

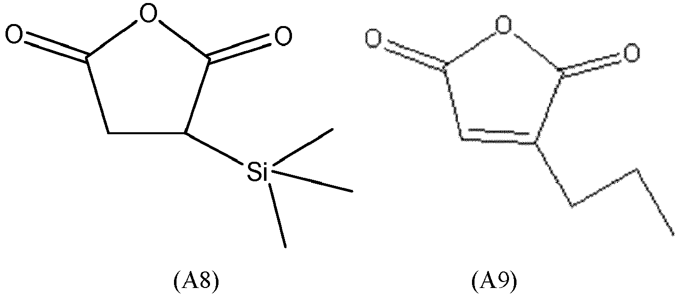

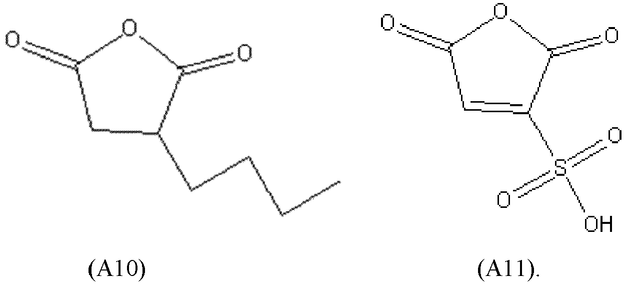

Optionally, the compound (A) is selected from one or more of the above compounds (A1), (A2), (A4)-(A6), (A8), (A9), and (A11).

Therefore, the ability of the compound (A) to capture the transition metal ions can be further improved, the film-forming ability of the compound (A) on the surface of the positive electrode is improved, and thus the protective effect of the compound (A) on the positive electrode material and the cycling life and storage performance of the secondary battery can be further improved.

In any embodiment, the content of the compound (A) in the electrolyte is 1 wt%-5 wt%, optionally 2 wt%-3 wt%. Therefore, in maintaining the protective effect of the compound (A) on the positive electrode material and the ionic conduction capacity of the electrolyte, the cycling life and storage performance of the secondary battery can be improved without increasing the internal resistance of the battery.

In any embodiment, the organic solvent is free of ethylene carbonate (EC). Therefore, the permeation corrosion of the solvent in the electrolyte into the positive electrode material can be further reduced, the collapse of the structure of the positive electrode material caused by the dissolution of the transition metal ions in the electrolyte can be further prevented, and thus the cycling life and storage performance of the battery can be further improved.

In any embodiment, the organic solvent contains one or more selected from a chain carbonate, a cyclic carbonate except ethylene carbonate, a carboxylate, and an ether solvent. Therefore, the electrolyte can be guaranteed to have sufficient ionic conduction capacity even when the content of ethylene carbonate is reduced, such that the cycling life and storage performance of the battery can be reliably improved.

In any embodiment, the content of the chain carbonate in the electrolyte is 40 wt%-90 wt%. Therefore, in maintaining the protective effect of the electrolyte on the positive electrode material and the ionic conduction capacity of the electrolyte, the cycling life and storage performance of the battery can be reliably improved.

In any embodiment, the chain carbonate is selected from dimethyl carbonate, diethyl carbonate, dipropyl carbonate, methyl ethyl carbonate, methyl propyl carbonate, ethyl propyl carbonate, and trifluoroethyl methyl carbonate; the cyclic carbonate except ethylene carbonate is selected from fluoroethylene carbonate; the carboxylate is selected from ethyl acetate, methyl acetate, and propyl acetate; and the ether solvent is selected from 1, 1,2,2-tetrafluoroethyl-2,2,2-trifluoroethyl ether, desflurane, and 2,2,2-trifluoroethyl-30,30,30,20,20-pentafluoropropyl ether. Therefore, the electrolyte has a good ionic conduction capacity and thus can reliably improve the cycling life and storage performance of the battery.

A second aspect of the present application provides a secondary battery, comprising an electrolyte of the first aspect of the present application.

A third aspect of the present application provides a battery module, comprising a secondary battery of the second aspect of the present application.

A fourth aspect of the present application provides a battery pack, comprising a battery module of the third aspect of the present application.

A fifth aspect of the present application provides a power consuming device, comprising at least one selected from the secondary battery of the second aspect of the present application, the battery module of the third aspect of the present application or the battery pack of the fourth aspect of the present application.

Therefore, the present application can provide a secondary battery, especially working at a high voltage (≥ 4.35V), with an improved cycling life and storage performance, and a battery module, a battery pack, and a power consuming device comprising the secondary battery.

### Brief Description of the Drawings

Fig. 1 is a schematic diagram of a secondary battery according to an embodiment of the present application.
Fig. 2 is an exploded view of a secondary battery according to an embodiment of the present application as shown in Fig.1.
Fig. 3 is a schematic diagram of a battery module according to an embodiment of the present application.
Fig. 4 is a schematic diagram of a battery pack according to an embodiment of the present application.
Fig. 5 is an exploded view of a battery pack according to an embodiment of the present application as shown in Fig. 4.
Fig. 6 is a schematic diagram of a power consuming device according to an embodiment of the present application.

### List of reference signs:

1 battery pack; 2 upper case body; 3 lower case body; 4 battery module; 5 secondary battery; 51 housing; 52 electrode assembly; and 53 top cover assembly.

### Detailed Description of Embodiments

Hereafter, embodiments of the electrolyte, secondary battery, battery module, battery pack, and electrical device of the present application are specifically disclosed in the detailed description with reference to the accompanying drawings as appropriate. However, unnecessary detailed illustrations may be omitted in some instances. For example, there are situations where detailed description of well-known items and repeated description of actually identical structures are omitted. This is to prevent the following description from being unnecessarily verbose, and facilitates understanding by those skilled in the art. Moreover, the accompanying drawings and the descriptions below are provided for enabling those skilled in the art to fully understand the present application, rather than limiting the subject matter disclosed in claims.

"Ranges" disclosed in the present application are defined in the form of lower and upper limits, a given range is defined by selection of a lower limit and an upper limit, and the selected lower and upper limits define the boundaries of the particular range. Ranges defined in this manner may be inclusive or exclusive, and may be arbitrarily combined, that is, any lower limit may be combined with any upper limit to form a range. For example, if the ranges of 60-120 and 80-110 are listed for a particular parameter, it should be understood that the ranges of 60-110 and 80-120 are also contemplated. Additionally, if minimum range values 1 and 2 are listed, and maximum range values 3, 4, and 5 are listed, the following ranges are all contemplated: 1-3, 1-4, 1-5, 2-3, 2-4 and 2-5. In the present application, unless stated otherwise, the numerical range "a-b" denotes an abbreviated representation of any combination of real numbers between a and b, where both a and b are real numbers. For example, the numerical range "0-5" means that all real numbers between "0-5" have been listed herein, and "0-5" is just an abbreviated representation of combinations of these numerical values. In addition, when a parameter is expressed as an integer of ≥ 2, it is equivalent to disclosing that the parameter is, for example, an integer of 2, 3, 4, 5, 6, 7, 8, 9, 10, 11, 12, and the like.

All the embodiments and optional embodiments of the present application can be combined with one another to form new technical solutions, unless otherwise stated.

All technical features and optional technical features of the present application can be combined with one another to form a new technical solution, unless otherwise stated.

Unless otherwise stated, all the steps of the present application can be performed sequentially or randomly, preferably sequentially. For example, the method including steps (a) and (b) indicates that the method may include steps (a) and (b) performed sequentially, and may also include steps (b) and (a) performed sequentially. For example, reference to "the method may further include step (c)" indicates that step (c) may be added to the method in any order, e.g., the method may include steps (a), (b) and (c), or steps (a), (c) and (b), or steps (c), (a) and (b), etc.

The terms "comprise" and "include" mentioned in the present application are open-ended or closed-ended, unless otherwise stated. For example, the terms "comprise" and "include" may mean that other components not listed may also be comprised or included, or only the listed components may be comprised or included.

In the present application, the term "or" is inclusive unless otherwise specified. For example, the phrase "A or B" means "A, B, or both A and B". More specifically, a condition "A or B" is satisfied by any one of the following: A is true (or present) and B is false (or not present); A is false (or not present) and B is true (or present); or both A and B are true (or present).

In the prior art, impurities such as hydrofluoric acid, etc., may be generated due to reaction of a lithium salt in the electrolyte and extremely trace water. The impurities may corrode a positive electrode material and dissolve active substances such as transition metals in the positive electrode material, thereby resulting in the collapse of the structure of the positive electrode material and directly reducing the storage performance and cycling life of the battery. In addition, when the battery operates in a high-voltage system, a redox reaction is more likely to occur on the surface of the positive electrode material, transition metal ions in the positive electrode material are rapidly dissolved out under the action of the electrolyte, and thus the electrochemical performance of the positive electrode material is severely restricted and battery safety problems such as explosion may be further caused.

The inventor of the present application has studied the electrolyte. On one hand, a specific additive is added to the electrolyte, and the additive can form a film on the surface of an electrode to protect the positive electrode material. On the other hand, the components of the electrolyte are adjusted, and the content of the components which can corrode the positive electrode material in the electrolyte is reduced. Therefore, the positive electrode material is synergistically protected from being corroded and dissolved out by the electrolyte, the cycling life and storage performance of the battery are improved, and particularly, when the battery is operated under a high voltage, the positive electrode material can also be protected, the storage performance of the secondary battery is stabilized, and the cycling life of the secondary battery is improved.

### [Electrolyte]

An embodiment of the present application provides an electrolyte, wherein the electrolyte comprises an organic solvent and one or more of a compound (A) represented by the following general formula (I) or general formula (II), the content of the compound (A) in the electrolyte is 0.01 wt%-5 wt% and the content of ethylene carbonate in the organic solvent is less than or equal to 3 wt%. 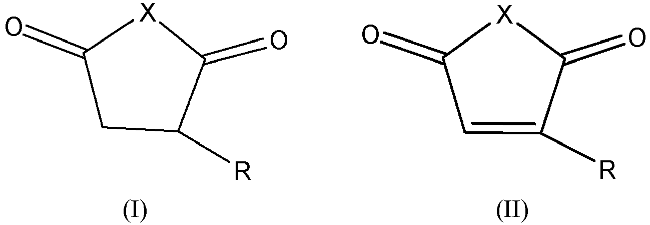 (In the general formula (I) or general formula (II), X is any one element selected from O, S, and N; when X is O, R comprises one or more of a cyano or isocyanate group, a trimethylsilyl, a straight-chain alkyl or branched-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; and when X is S or N, R comprises one or more of a hydrogen atom, a cyano or isocyanate group, a trimethylsilyl, a straight-chain alkyl or branched-chain alkyl with 1-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen.)

The applicant has unexpectedly found that: in the present application, by containing the specific compound (A) in the electrolyte in the above specific range and reducing the content of the commonly used ethylene carbonate in the organic solvent, the erosive power of the electrolyte to the positive electrode material can be reduced, a protective film can also be formed on the surface of the positive electrode material, and thus the positive electrode material can be reliably protected from being eroded and dissolved by the electrolyte and the cycling life and storage performance of the secondary battery can be improved.

Although the mechanism is not clearly understood, it is speculated by the applicant of the present invention as follows. The specific compound (A) has a complexing ability to the transition metals, and thus can capture the transition metal ions dissolved in the electrolyte to prevent the transition metals from being dissolved out by the electrolyte. In addition, the specific compound (A) has a certain adsorption effect on the positive electrode material and can form a film on the surface of the positive electrode material to protect the positive electrode material from being corroded by the electrolyte.

In addition, in the prior art, an organic solvent containing a large amount of ethylene carbonate (EC) has been always used in the electrolyte to improve the conductivity of the electrolyte. However, during the battery cycling or storage, the ethylene carbonate solvent continuously diffuses and penetrates through an SEI film on the surface of the positive electrode material, directly contacts with the surface of the positive electrode active material particle, and is subjected to oxidative decomposition. In addition, in the oxidative decomposition, side reactions such as gas generation, oxygen release, and generation of acidic substances are often accompanied. The generated acidic substances further corrode the positive electrode material, thereby further dissolving out the transition metals and accelerating the cell capacity attenuation. Therefore, the corrosion of the solvent in the electrolyte to the positive electrode material can be reduced by reducing the content of the ethylene carbonate in the electrolyte and the protective effect of the electrolyte to the positive electrode material is further enhanced.

In some embodiments, in the above general formula (I) or general formula (II), when X is O, R comprises one or more of a cyano or isocyanate group, a trimethylsilyl, a straight-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; and when X is S or N, R comprises one or more of a cyano or isocyanate group, a trimethylsilyl, a hydrogen atom, a straight-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen. Optionally, when X is O, R comprises one or more of a cyano or isocyanate group, a trimethylsilyl, a straight-chain alkyl with 2-3 carbon atoms, a straight-chain alkyl with 1-3 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; and when X is S or N, R comprises one or more of a cyano or isocyanate group, a trimethylsilyl, a hydrogen atom, a straight-chain alkyl with 2-3 carbon atoms, a straight-chain alkyl with 1-3 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen.

By selecting proper X and R, the complexing ability of the compound (A) to the transition metal ions can be enhanced, the adsorbability of the compound (A) to the surface of the positive electrode material can be improved, and thus the capturing ability of the compound (A) to the transition metal ions can be further improved, the film-forming ability of the compound (A) on the surface of the positive electrode can be improved, the transition metal ions can be effectively prevented from being dissolved in the electrolyte, and the positive electrode material can be protected from being corroded by the electrolyte.

In some embodiments, the compound (A) is selected from one or more of the following compounds (A1)-(A12). Optionally, the compound (A) is selected from one or more of the compounds (A1), (A2), (A4)-(A6), (A8), (A9), and (A11).

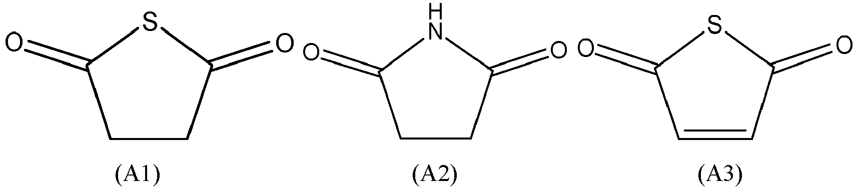

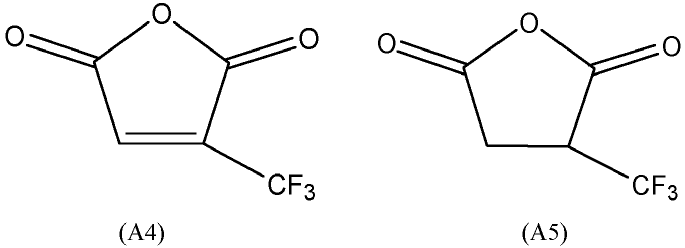

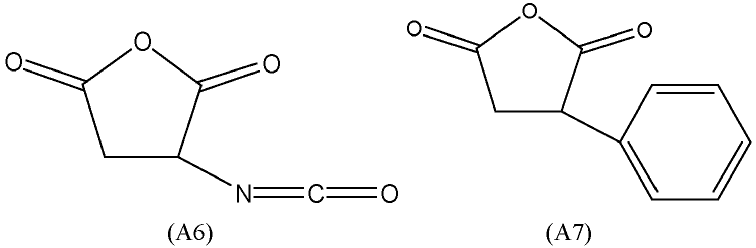

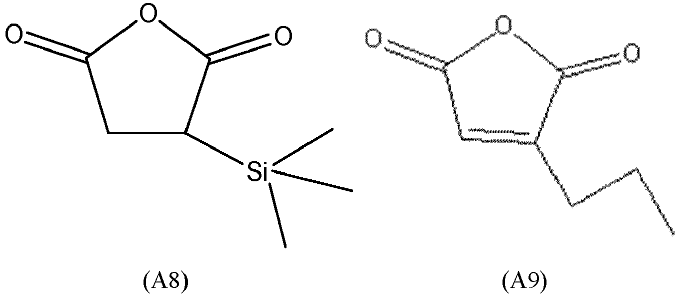

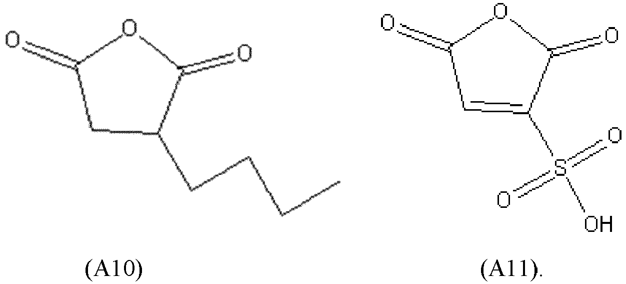

Therefore, the compound (A) can efficiently capture the transition metal ions dissolved out in the positive electrode material, easily form a film on the surface of the positive electrode material, and thus can protect the positive electrode material from being eroded by the electrolyte.

In some embodiments, the content of the compound (A) in the electrolyte is 1 wt%-5 wt%, optionally 2 wt%-3 wt%. If the content of the compound (A) in the electrolyte is too small, a good interfacial film cannot be formed on the surface of the positive electrode and it is difficult to prevent the decomposition of the solvent and the dissolution of the transition metals at a high voltage. If the content of the compound (A) in the electrolyte is too large, the viscosity of the electrolyte increases, a transfer resistance of lithium ions increases, a too thick interfacial film is formed on the surface of the positive electrode, and thus the film resistance and charge transfer resistance of the secondary battery are too large.

In some embodiments, the organic solvent is substantially free of ethylene carbonate. Therefore, an influence of the ethylene carbonate on the positive electrode material can be completely eliminated and the transition metals are prevented from being corroded and dissolved out.

In some embodiments, the organic solvent contains one or more selected from a chain carbonate, a cyclic carbonate other than ethylene carbonate, a carboxylate, and an ether solvent. Therefore, the function of the commonly used ethylene carbonate in the prior art can be replaced, thus the corrosion of the electrolyte on the positive electrode material is reduced, the conductivity of the electrolyte can also be ensured, the normal charging/discharging of the secondary battery can be ensured, and thus the cycling life and storage performance of the battery can be reliably improved.

In some embodiments, the content of the chain carbonate in the electrolyte is 40 wt%-90 wt%. Therefore, in maintaining the protective effect of the electrolyte on the positive electrode material and the ionic conduction capacity of the electrolyte, the cycling life and storage performance of the battery can be reliably improved.

In some embodiments, the chain carbonate is selected from dimethyl carbonate, diethyl carbonate, dipropyl carbonate, methyl ethyl carbonate, methyl propyl carbonate, ethyl propyl carbonate, and trifluoroethyl methyl carbonate; the cyclic carbonate other than ethylene carbonate is selected from fluoroethylene carbonate; the carboxylate is selected from ethyl acetate, methyl acetate, and propyl acetate; and the ether solvent is selected from 1, 1,2,2-tetrafluoroethyl-2,2,2-trifluoroethyl ether, desflurane, and 2,2,2-trifluoroethyl-30,30,30,20,20-pentafluoropropyl ether. Therefore, the electrolyte has a good ionic conduction capacity and thus can reliably improve the cycling life and storage performance of the battery.

In some embodiments, the content of the organic solvent in the electrolyte is 60 wt%-98 wt%.

In some embodiments, the organic solvent can further include any one selected from 1,4-butyrolactone, sulfolane, dimethyl sulfone, methyl ethyl sulfone, diethylsulfone, etc.

In some embodiments, the electrolyte further includes an electrolyte salt, wherein the electrolyte salt may be selected from at least one of lithium hexafluorophosphate, lithium tetrafluoroborate, lithium perchlorate, lithium hexafluoroarsenate, lithium bisfluorosulfonimide, lithium bistrifluoromethanesulfonimide, lithium trifluoromethanesulfonate, lithium difluorophosphate, lithium difluorooxalate borate, lithium dioxalate borate, lithium difluorodioxalate phosphate, and lithium tetrafluorooxalate phosphate.

In some embodiments, the electrolyte may optionally comprise a negative electrode film-forming additive and other additives, and may further comprise an additive capable of improving certain performances of the battery, such as an additive capable of improving an overcharge performance of the battery, an additive capable of improving a high-or low-temperature performance of the battery, and the like.

In addition, the secondary battery, battery module, battery pack, and power consuming device of the present application will be described below by appropriately referring to the accompanying drawings.

### [Secondary battery]

In one embodiment of the present application, a secondary battery is provided.

Generally, a secondary battery comprises a positive electrode plate, a negative electrode plate, an electrolyte, and a separator. During a charge/discharge process of the battery, active ions are intercalated and de-intercalated back and forth between the positive electrode plate and the negative electrode plate. The electrolyte is between the positive electrode plate and the negative electrode plate and functions for conducting ions. The separator is provided between the positive electrode plate and the negative electrode plate, and mainly prevents the positive and negative electrodes from short-circuiting and enables ions to pass through.

The electrolyte in the secondary battery of the present embodiment is the electrolyte provided in the above embodiment.

### [Positive electrode plate]

The positive electrode plate comprises a positive current collector and a positive film layer provided on at least one surface of the positive current collector, and the positive film layer comprises a positive active material.

As an example, the positive electrode current collector has two surfaces opposite in its own thickness direction, and the positive electrode film layer is provided on either or both of opposite surfaces of the positive electrode current collector.

In some embodiments, the positive electrode current collector can be a metal foil or a composite current collector. For example, as a metal foil, an aluminum foil can be used. The composite current collector may comprise a polymer material base layer and a metal layer formed on at least one surface of the polymer material base layer. The composite current collector can be formed by forming a metal material (aluminum, an aluminum alloy, nickel, a nickel alloy, titanium, a titanium alloy, silver and a silver alloy, etc.) on a polymer material substrate (such as polypropylene (PP), polyethylene terephthalate (PET), polybutylene terephthalate (PBT), polystyrene (PS), polyethylene (PE), etc.).

In some embodiments, the positive electrode active material may be a positive active material known in the art for batteries. As an example, the positive electrode active material may comprise at least one of the following materials: lithium-containing phosphates of an olivine structure, lithium transition metal oxides and their respective modified compounds. However, the present application is not limited to these materials, and other conventional materials that can be used as positive electrode active materials for batteries may also be used. These positive electrode active materials may be used alone or in combination of two or more. Herein, examples of lithium transition metal oxides may include, but are not limited to, at least one of lithium cobalt oxide (e.g. LiCoO₂), lithium nickel oxide (e.g. LiNiO₂), lithium manganese oxide (e.g. LiMnO₂, LiMn₂O₄), lithium nickel cobalt oxide, lithium manganese cobalt oxide, lithium nickel manganese oxide, lithium nickel cobalt manganese oxide (e.g. LiNi_{1/3}Co_{1/3}Mn_{1/3}O₂ (also referred to as NCM₃₃₃), LiNi_{0.5}Co_{0.2}Mn_{0.3}O₂ (also referred to as NCM₅₂₃), LiNi_{0.5}Co_{0.25}Mn_{0.25}O₂ (also referred to as NCM₂₁₁), LiNi_{0.6}Co_{0.2}Mn_{0.2}O₂ (also referred to as NCM6₂₂), LiNi_{0.8}Co_{0.1}Mn_{0.1}O₂ (also referred to as NCM₈₁₁)), lithium nickel cobalt aluminum oxide (e.g. LiNi_{0.85}Co_{0.15}Al_{0.05}O₂), and modified compounds thereof, and the like. Examples of lithium-containing phosphates of olivine structure may include, but are not limited to, at least one of lithium iron phosphate (e.g. LiFePO₄ (also referred to as LFP)), lithium iron phosphate and carbon composites, lithium manganese phosphate (e.g. LiMnPOa), lithium manganese phosphate and carbon composites, lithium iron manganese phosphate, and lithium iron manganese phosphate and carbon composites.

In some embodiments, the positive electrode film layer may optionally comprise a binder. As an example, the binder may include at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, and fluorine-containing acrylate resin.

In some embodiments, the positive electrode film layer also optionally comprises a conductive agent. As an example, the conductive agent may include at least one of superconducting carbon, acetylene black, carbon black, Ketjen black, carbon dots, carbon nanotubes, graphene, and carbon nanofibers.

In some embodiments, the positive electrode plate can be prepared as follows: dispersing the above-described components for preparing the positive electrode plate, such as a positive active material, a conductive agent, a binder and any other components, in a solvent (e.g., N-methylpyrrolidone) to form a positive electrode slurry; and coating the positive electrode current collector with the positive electrode slurry, followed by the procedures such as drying and cold pressing, so as to obtain the positive electrode plate.

### [Negative electrode plate]

The negative electrode plate comprises a negative electrode current collector and a negative electrode film layer provided on at least one surface of the negative electrode current collector, and the negative electrode film layer comprises a negative electrode active material.

As an example, the negative electrode current collector has two surfaces opposite in its own thickness direction, and the negative electrode film layer is provided on either or both of the two opposite surfaces of the negative electrode current collector.

In some embodiments, the negative electrode current collector can be a metal foil or a composite current collector. For example, as a metal foil, a copper foil can be used. The composite current collector may comprise a polymer material base layer and a metal layer formed on at least one surface of the polymer material substrate. The composite current collector can be formed by forming a metal material (copper, a copper alloy, nickel, a nickel alloy, titanium, a titanium alloy, silver and a silver alloy, etc.) on a polymer material substrate (e.g., polypropylene (PP), polyethylene terephthalate (PET), polybutylene terephthalate (PBT), polystyrene (PS), polyethylene (PE), etc.).

In some embodiments, the negative electrode active material can be a negative electrode active material known in the art for batteries. As an example, the negative electrode active material may include at least one of the following materials: synthetic graphite, natural graphite, soft carbon, hard carbon, a silicon-based material, a tin-based material and lithium titanate, etc. The silicon-based material may be selected from at least one of elemental silicon, silicon oxides, silicon carbon composites, silicon nitrogen composites and silicon alloys. The tin-based material may be selected from at least one of elemental tin, tin oxides, and tin alloys. However, the present application is not limited to these materials, and other conventional materials that can be used as negative electrode active materials for batteries can also be used. These negative electrode active materials may be used alone or in combination of two or more.

In some embodiments, the negative electrode film layer may optionally comprise a binder. The binder may be selected from at least one of a butadiene styrene rubber (SBR), polyacrylic acid (PAA), sodium polyacrylate (PAAS), polyacrylamide (PAM), polyvinyl alcohol (PVA), sodium alginate (SA), polymethacrylic acid (PMAA) and carboxymethyl chitosan (CMCS).

In some embodiments, the negative electrode film layer may optionally comprise a conductive agent. The conductive agent may be selected from at least one of superconductive carbon, acetylene black, carbon black, ketjenblack, carbon dots, carbon nanotubes, graphene, and carbon nanofibers.

In some embodiments, the negative electrode film layer may optionally comprise other auxiliary agents, such as a thickener (e.g. sodium carboxymethyl cellulose (CMC-Na)) and the like.

In some embodiments, the negative electrode plate can be prepared as follows: dispersing the above-mentioned components for preparing the negative electrode plate, such as a negative electrode active material, a conductive agent, a binder and any other components, in a solvent (e.g. deionized water) to form a negative electrode slurry; and coating a negative electrode current collector with the negative electrode slurry, followed by procedures such as drying and cold pressing, so as to obtain the negative electrode plate.

### [Separator]

In some embodiments, the secondary battery further comprises a separator. The type of the separator is not particularly limited in the present application, and any well-known porous-structure separator with good chemical stability and mechanical stability may be selected.

In some embodiments, the material of the separator may be selected from at least one of glass fibers, non-woven fabrics, polyethylene, polypropylene and polyvinylidene fluoride. The separator may be a single-layer film or a multi-layer composite film, and is not limited particularly. When the separator is a multi-layer composite film, the materials in the respective layers may be same or different, which is not limited particularly.
In some embodiments, the positive electrode plate, the negative electrode plate and the separator can be made into the electrode assembly by a winding process or a stacking process.

In some embodiments, the secondary battery may comprise an outer package. The outer package can be used to package the electrode assembly and electrolyte as described above.

In some implementations, the outer package of the secondary battery may be a hard shell, such as a hard plastic shell, an aluminum shell, or a steel shell. The outer package of the secondary battery may also be a soft bag, such as a pouch-type soft bag. The material of the soft bag may be plastics, and the examples of plastics may include polypropylene, polybutylene terephthalate, and polybutylene succinate, etc.

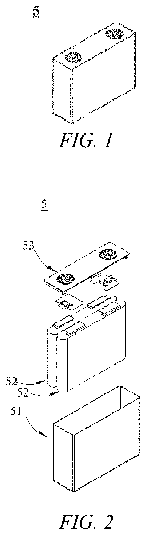

The shape of the secondary battery is not particularly limited in the present application, and may be cylindrical, square or of any other shape. For example, Fig. 1 shows a secondary battery 5 with a square structure as an example.

In some embodiments, referring to Fig. 2, an outer package may comprise a housing 51 and a cover plate 53. Herein, the housing 51 may comprise a bottom plate and side plates connected to the bottom plate, and the bottom plate and the side plates enclose to form an accommodating cavity. The housing 51 has an opening in communication with the accommodating cavity, and the cover plate 53 can cover the opening to close the accommodating cavity. The positive electrode plate, the negative electrode plate and the separator can be subjected to a winding process or a stacking process to form an electrode assembly 52. The electrode assembly 52 is encapsulated in the accommodating cavity. The electrolyte infiltrates the electrode assembly 52. The number of the electrode assemblies 52 contained in the secondary battery 5 may be one or more, and can be selected by those skilled in the art according to actual requirements.

In some embodiments, the secondary battery can be assembled into a battery module, and the number of the secondary batteries contained in the battery module may be one or more, and the specific number can be selected by those skilled in the art according to the application and capacity of the battery module.

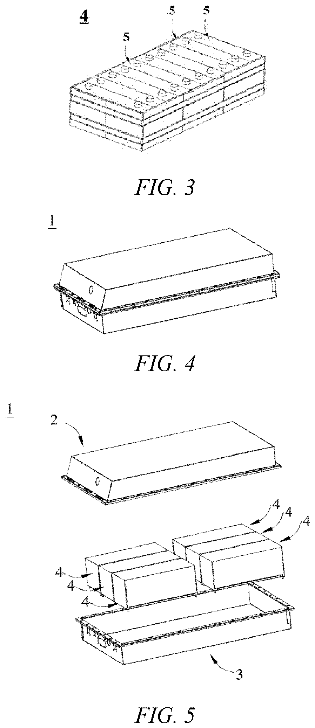

Fig. 3 shows a battery module 4 as an example. Referring to Fig. 3, in the battery module 4, a plurality of the secondary batteries 5 may be arranged in sequence in the length direction of the battery module 4. Apparently, the secondary batteries may also be arranged in any other manner. Furthermore, the plurality of the secondary batteries 5 may be fixed by fasteners.

Optionally, the battery module 4 may also comprise a housing with an accommodating space, and a plurality of the secondary batteries 5 are accommodated in the accommodating space.

In some embodiments, the above battery module may also be assembled into a battery pack, the number of the battery modules contained in the battery pack may be one or more, and the specific number can be selected by those skilled in the art according to the application and capacity of the battery pack.

Fig. 4 and Fig. 5 show a battery pack 1 as an example. Referring to Fig. 4 and Fig. 5, the battery pack 1 may comprise a battery case and a plurality of the battery modules 4 provided in the battery box. The battery case comprises an upper case body 2 and a lower case body 3, wherein the upper case body 2 can cover the lower case body 3 to form a closed space for accommodating the battery modules 4. A plurality of the battery modules 4 may be arranged in the battery case in any manner.

In addition, the present application further provides a power consuming device. The power consuming device comprises at least one of the secondary battery, battery module, or battery pack provided by the present application. The secondary battery, battery module or battery pack can be used as a power source of the power consuming device or as an energy storage unit of the power consuming device. The power consuming device may include a mobile device (e.g., a mobile phone, a laptop computer, etc.), an electric vehicle (e.g., a pure electric vehicle, a hybrid electric vehicle, a plug-in hybrid electric vehicle, an electric bicycle, an electric scooter, an electric golf cart, an electric truck), an electric train, a ship, and a satellite, an energy storage system, and the like, but is not limited thereto.

As for the power consuming device, the secondary battery, battery module or battery pack can be selected according to the usage requirements thereof.

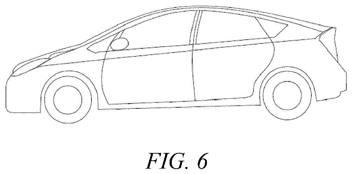

Fig. 6 shows a power consuming device as an example. The power consuming device may be a pure electric vehicle, a hybrid electric vehicle, a plug-in hybrid electric vehicle or the like. In order to meet the requirements of the power consuming device for a high power and a high energy density of a secondary battery, a battery pack or a battery module may be used.

As another example, the device may be a mobile phone, a tablet computer, a laptop computer, etc. The device is generally required to be thin and light, and may use the secondary battery as a power supply.

### Examples

Hereinafter, the examples of the present application will be explained. The examples described below are exemplary and are merely for explaining the present application, and should not be construed as limiting the present application. The examples in which techniques or conditions are not specified are based on the techniques or conditions described in documents in the art or according to the product introduction. If not marked with the manufacturer, the used reagents or instruments are commercially available common products.

### Examples 1-19 and comparative examples 1-8

### (1) Preparation of electrolyte

The electrolytes of examples 1-19 and comparative examples 1-8 are prepared according to the following method:
In an argon atmosphere glove box with a water content < 10 ppm, methyl ethyl carbonate (EMC) of a chain carbonate is used as a main solvent, and each auxiliary solvent is mixed with the methyl ethyl carbonate (EMC) as a main solvent at the weight ratio of the auxiliary solvent to the main solvent (auxiliary solvent: main solvent (EMC)) shown in Table 1 to obtain a mixed solvent. Then the compound (A) shown in Table 1 and a fully dried lithium salt LiPF₆ are dissolved in the above mixed solvent to a concentration of LiPF₆ at 1 mol/L, and the materials are stirred uniformly to obtain an electrolyte. The weight percentage (wt%) of the content of the compound (A) indicated in Table 1 is a weight percentage (wt%) relative to the electrolyte.

### (2) Preparation of secondary battery

The secondary batteries of examples 1-19 and comparative examples 1-8 are prepared according to the following method.

### [Preparation of negative electrode plate]

A negative electrode active material of graphite, a conductive agent of acetylene black, a binder of a styrene-butadiene rubber (SBR), and a thickener of sodium carboxymethyl cellulose (CMC) are mixed at a weight ratio of 95 : 2 : 2 : 1, deionized water is added, and the materials are fully stirred and mixed uniformly to form a uniform negative electrode slurry. The negative electrode slurry is coated onto a negative electrode current collector of a copper foil, followed by drying and cold pressing, to obtain a negative electrode plate.

### [Preparation of positive electrode plate]

A positive electrode active material of NCM211 (LiNi_{0.5}Co_{0.25}Mn_{0.25}O₂), a conductive agent of acetylene black, and a binder of polyvinylidene fluoride are mixed at a weight ratio of 97 : 2 : 1, a solvent of N-methylpyrrolidone is added, and the materials are fully stirred and mixed uniformly to form a uniform positive electrode slurry. The slurry is coated onto a positive electrode current collector of an aluminum foil, followed by drying and cold pressing, to obtain a positive electrode plate.

### [Preparation of battery]

The positive electrode plate, separator, and negative electrode plate are stacked in sequence to such that the separator is located between the positive and negative electrode plates to functions for separation, and then wound to obtain a bare cell. The bare cell is placed in an outer packaging foil, the electrolyte prepared in (1) is injected into the dried bare cell, followed by vacuum packaging, standing, forming, shaping and other processes to obtain a secondary battery.

**[Table 1]**

| Sample | Compound (A) | Organic solvent | | |
|---|---|---|---|---|
| | | Types of auxiliary solvents | Auxiliary solvents: main solvent (EMC) | Content of EC (wt%) |
| Example 1 | 0.1 wt% A4 | - | 0 : 100 | 0 |
| Example 2 | 1 wt% A4 | - | 0 : 100 | 0 |
| Example 3 | 2 wt% A4 | - | 0 : 100 | 0 |
| Example 4 | 3 wt% A4 | - | 0 : 100 | 0 |
| Example 5 | 5 wt% A4 | - | 0 : 100 | 0 |
| Example 6 | 2 wt% A1 | - | 0 : 100 | 0 |
| Example 7 | 2 wt% A2 | - | 0 : 100 | 0 |
| Example 8 | 2 wt% A5 | - | 0 : 100 | 0 |
| Example 9 | 2 wt% A6 | - | 0 : 100 | 0 |
| Example 10 | 2 wt% A8 | - | 0 : 100 | 0 |
| Example 11 | 2 wt% A9 | - | 0 : 100 | 0 |
| Example 12 | 2 wt% A11 | - | 0: 100 | 0 |
| Example 13 | 1 wt% A4 + 1 wt% A6 | - | 0 : 100 | 0 |
| Example 14 | 1 wt% A4 + 1 wt% A1 1 | - | 0 : 100 | 0 |
| Example 15 | 2 wt% A4 | FEC | 30 : 70 | 0 |
| Example 16 | 2 wt% A4 | D-FEC | 30: 70 | 0 |
| Example 17 | 2 wt% A4 | FEMC | 30 : 70 | 0 |
| Example 18 | 2 wt% A4 | HFE | 30 : 70 | 0 |
| Example 19 | 2 wt% A4 | EC | 3 : 97 | 3 |
| Comparative example 1 | / | - | 0 : 100 | 0 |
| Comparative example 2 | 0.005 wt% A4 | - | 0: 100 | 0 |
| Comparative example 3 | 6 wt% A4 | - | 0 : 100 | 0 |
| Comparative example 4 | / | EC | 3 : 97 | 3 |
| Comparative example 5 | / | EC | 5 : 95 | 5 |
| Comparative example 6 | / | EC | 30: 70 | 30 |
| Comparative example 7 | 2 wt% A4 | EC | 30: 70 | 30 |
| Comparative example 8 | 2 wt% A4 | EC | 5 : 95 | 5 |

| | | | | |
|---|---|---|---|---|
| Note: In Table 1, "j" indicates that no compound (A) of any kind is added. "-" means that EMC is used as a solvent instead of an auxiliary solvent. The EC content is expressed as a weight percentage (wt%) relative to the organic solvent. | | | | |

High-temperature cycling performance and high-temperature storage performance of the secondary batteries obtained in examples 1-19 and comparative examples 1-8 under high voltage working conditions are tested according to the following methods. The results are shown in Table 2.

### (1) Test of high-temperature cycling performance of secondary battery

After the secondary batteries are prepared, at 45°C, the secondary batteries are firstly charged to 4.5 V at a constant current of 0.5 C, then further charged to 0.025 C at a constant voltage of 4.5 V, and then discharged to 3.0 V at a constant current of 0.5 C. This is a charge/discharge cycle, and the discharge capacity of this process is used as the discharge capacity of the 1st cycle. The secondary batteries are subjected to the charge/discharge cycling test according to the method described above, and the discharge capacity in the 100th cycle is measured. A capacity retention rate (%) of the secondary batteries after the high temperature cycling is calculated according to the following formula. The results are shown in Table 2.

Capacity retention rate of secondary battery after high-temperature cycling (%) = [discharge capacity of the 100th cycle/discharge capacity of the 1st cycle] × 100%.

### (2) Test of high-temperature storage performance of secondary battery

After the secondary batteries are prepared, at 25°C, the secondary batteries are firstly charged to 4.5 V at a constant current of 0.5 C and then further charged to 0.025 C at a constant voltage of 4.5 V. Then the volume of the secondary batteries is measured in deionized water using a displacement method as an initial volume of the secondary batteries (i.e., the volume of the secondary batteries before high-temperature storage). Then the surfaces of the secondary batteries are dried, the secondary batteries are stored at 60°C for 30 days. After the storage is ended, the volume of the secondary batteries is tested as the volume of the secondary batteries after the high-temperature storage. A volume expansion rate (%) of the secondary batteries after the high-temperature storage is calculated according to the following formula. The results are shown in Table 2.

Volume expansion rate of secondary battery after high-temperature storage (%) = [(volume of secondary battery after high-temperature storage-initial volume of secondary battery)/initial volume of secondary battery] × 100%.

**[Table 2]**

| Sample | Capacity retention rate after high-temperature cycling (%) | Volume expansion rate after high-temperature storage (%) |
|---|---|---|
| Example 1 | 65 | 97 |
| Example 2 | 77 | 33 |
| Example 3 | 93 | 13 |
| Example 4 | 87 | 12 |
| Example 5 | 70 | 9 |
| Example 6 | 91 | 12 |
| Example 7 | 87 | 15 |
| Example 8 | 90 | 10 |
| Example 9 | 89 | 10 |
| Example 10 | 87 | 8 |
| Example 11 | 87 | 12 |
| Example 12 | 88 | 10 |
| Example 13 | 94 | 11 |
| Example 14 | 90 | 12 |
| Example 15 | 93 | 8 |
| Example 16 | 92 | 10 |
| Example 17 | 91 | 12 |
| Example 18 | 95 | 13 |
| Example 19 | 85 | 16 |
| Comparative example 1 | 37 | 122 |
| Comparative example 2 | 38 | 118 |
| Comparative example 3 | 55 | 5 |
| Comparative example 4 | 35 | 138 |
| Comparative example 5 | 32 | 183 |
| Comparative example 6 | 25 | 228 |
| Comparative example 7 | 81 | 33 |
| Comparative example 8 | 79 | 28 |

As can be seen from comparisons of examples 1-5 with comparative examples 1-3, in the case of not using ethylene carbonate (EC) as a solvent, the secondary batteries of examples 1-5 of the present invention containing an appropriate amount of the compound (A4) have a better high-temperature cycling performance and a high-temperature storage performance, i.e., a higher capacity retention rate after high-temperature cycling and a lower volume expansion rate after high-temperature storage, at high-pressure (4.5 V) operating conditions compared with the secondary battery of comparative example 1 not containing the compound (A). This is because the compound (A4) can protect the positive electrode by forming a film and capture transition metal ions dissolved in the electrolyte. However, when the content of the compound (A4) is more than 5 wt% (comparative example 3), the capacity retention rate after the high-temperature cycling is rather deteriorated. This is probably because the compound 1 is in a too large proportion in the organic solvent, increases the viscosity of the electrolyte, causes an increased transfer resistance of lithium ions, and also forms an excessively thick interfacial film on the surface of the positive electrode, resulting in a too large film resistance and a charge transfer resistance of the secondary battery and affecting the cycling performance of the secondary battery. When the weight percentage of the compound (A4) in the electrolyte is as low as 0.005 wt% (comparative example 2), since the content of the compound (A4) is too small, a good interface film cannot be efficiently formed on the surface of the positive electrode, thus it is difficult to prevent the dissolution of the transition metals in the positive electrode, and the performance of the secondary battery is not obviously improved.

In addition, from comparisons of examples 6-12 with comparative example 1, the high-temperature cycling performance and the high-temperature storage performance of the secondary batteries can be improved by using one or two or more of a plurality of the compound (A) corresponding to the general formula (I) or (II) as an additive. The resulting secondary batteries can obtain a higher capacity retention rate after the high-temperature cycling and a lower volume expansion rate after the high-temperature storage under high-pressure (4.5 V) operating conditions. This is because these compounds (A) have a relatively strong complexing ability on the transition metals. In addition, substituent groups such as F, trimethylsilyl, etc., of some other compounds (A) also have a certain adsorption effect on the positive electrode material. Therefore, the compounds (A) can functions for forming a film on the surface of the positive electrode and capturing the transition metals dissolved in the electrolyte required by the present application.

In addition, when examples 3 and 15-19 are compared with comparative examples 7 and 8, it is found that when an equal amount of the compound (A) is added, the high-temperature cycling performance and the high-temperature storage performance of the secondary batteries obtained by using an electrolyte of an organic solvent containing 5 wt% or more of ethylene carbonate (EC) are significantly deteriorated. Such a tendency can be observed also in comparisons of comparative example 1 with comparative examples 4-6 in the case of not containing compound (A). This is because when there is more EC in the organic solvent, EC will continuously diffuse and penetrate through an SEI film on the surface of the positive electrode material, directly contacts with the surface of the positive electrode active material particle, and is thus subjected to oxidative decomposition. In addition, in the oxidative decomposition, side reactions such as gas generation, oxygen release, and generation of acidic substances are accompanied. The generated acidic substances further corrode the positive electrode material, thereby further dissolving out the transition metals and accelerating the cell capacity attenuation. Therefore, the corrosion of the electrolyte to the positive electrode material can be reduced by reducing the content of EC in the electrolyte and the high-temperature cycling performance and the high-temperature storage performance of the secondary batteries can be improved. Furthermore, from comparisons between example 3 and example 19, it can be seen that the erosion effect of the EC and the side effect of gas generation can be substantially eliminated by controlling the EC percentage in the organic solvent to 3 wt% or less. Therefore, by further controlling the content of ethylene carbonate contained in the organic solvent of the electrolyte, the protection of the electrolyte on the positive electrode material can be further strengthened, such that the high-temperature cycling performance and high-temperature storage performance of the secondary batteries can be reliably improved.

It should be noted that the present application is not limited to the above embodiments. The above embodiments are exemplary only, and any embodiment that has substantially same constitutions as the technical ideas and has the same effects within the scope of the technical solution of the present application falls within the technical scope of the present application. In addition, without departing from the gist of the present application, various modifications that can be conceived by those skilled in the art to the embodiments, and other modes constructed by combining some of the constituent elements of the embodiments also fall within the scope of the present application.

## Claims

1. An electrolyte, wherein
the electrolyte comprises an organic solvent and one or more of a compound (A) represented by the following general formula (I) or general formula (II), 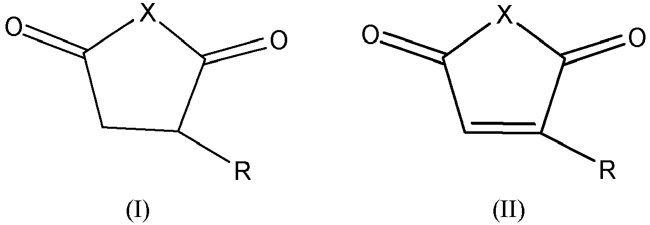
in the general formula (I) or general formula (II),
X is any one element selected from O, S, and N,
when X is O, R comprises one or more of a cyano or isocyanate group, a trimethylsilyl, a straight-chain alkyl or branched-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; when X is S or N, R comprises one or more of a hydrogen atom, a cyano or isocyanate group, a trimethylsilyl, a straight-chain alkyl or branched-chain alkyl with 1-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen;
a content of the compound (A) in the electrolyte is 0.01 wt%-5 wt%; and
a content of ethylene carbonate in the organic solvent is less than or equal to 3 wt%.

2. The electrolyte according to claim 1, wherein
in the general formula (I) or general formula (II),
when X is O, R comprises one or more of a cyano or isocyanate group, a trimethylsilyl, a straight-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; when X is S or N, R comprises one or more of a cyano or isocyanate group, a trimethylsilyl, a hydrogen atom, a straight-chain alkyl with 2-4 carbon atoms, a straight-chain alkyl with 1-4 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen;
optionally, when X is O, R comprises one or more of a cyano or isocyanate group, a trimethylsilyl, a straight-chain alkyl with 2-3 carbon atoms, a straight-chain alkyl with 1-3 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen; and when X is S or N, R comprises one or more of a cyano or isocyanate group, a trimethylsilyl, a hydrogen atom, a straight-chain alkyl with 2-3 carbon atoms, a straight-chain alkyl with 1-3 carbon atoms and with parts of hydrogens or all hydrogens replaced by a halogen, a phenyl or benzyl with parts of hydrogens or all hydrogens replaced by a halogen, and a sulfonyl or sulfonic group with parts of hydrogens or all hydrogens replaced by a halogen.

3. The electrolyte according to claim 1 or 2, wherein
the compound (A) is selected from one or more of the following compounds (A1)-(A12): 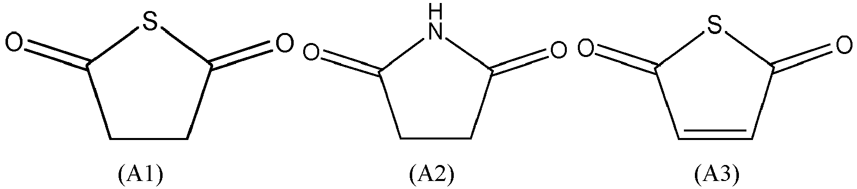 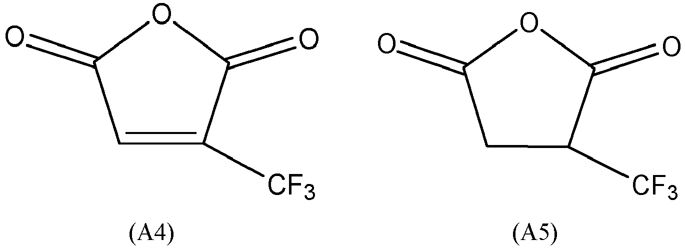 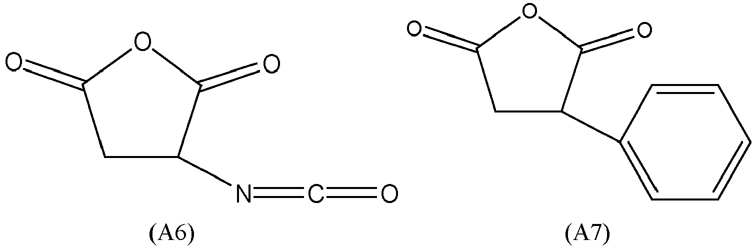 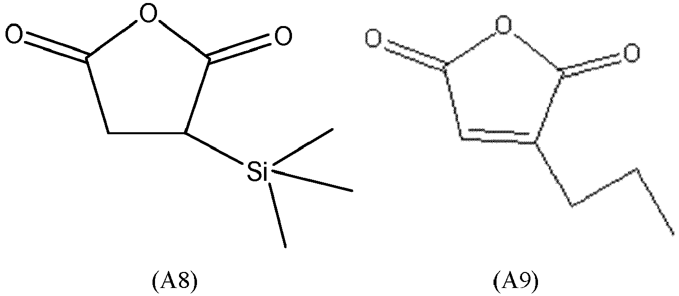 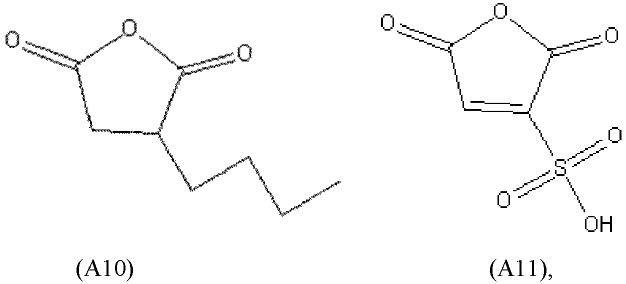
optionally, the compound (A) is selected from one or more of the above compounds (A1), (A2), (A4)-(A6), (A8), (A9), and (A11).

4. The electrolyte according to any one of claims 1-3, wherein
the content of the compound (A) in the electrolyte is 1 wt%-5 wt%, optionally 2 wt%-3 wt%.

5. The electrolyte according to any one of claims 1-4, wherein
the organic solvent is free of ethylene carbonate.

6. The electrolyte according to any one of claims 1-5, wherein
the organic solvent contains one or more selected from a chain carbonate, a cyclic carbonate other than ethylene carbonate, a carboxylate, and an ether solvent.

7. The electrolyte according to claim 6, wherein
a content of the chain carbonate in the electrolyte is 40 wt%-90 wt%.

8. The electrolyte according to claim 6 or 7, wherein
the chain carbonate is selected from dimethyl carbonate, diethyl carbonate, dipropyl carbonate, methyl ethyl carbonate, methyl propyl carbonate, ethyl propyl carbonate, and trifluoroethyl methyl carbonate; the cyclic carbonate other than ethylene carbonate is selected from fluoroethylene carbonate; the carboxylate is selected from ethyl acetate, methyl acetate, and propyl acetate; and the ether solvent is selected from 1, 1,2,2-tetrafluoroethyl-2,2,2-trifluoroethyl ether, desflurane, and 2,2,2-trifluoroethyl-30,30,30,20,20-pentafluoropropyl ether.

9. The electrolyte according to any one of claims 1-8, wherein
a content of the organic solvent in the electrolyte is 60 wt%-98 wt%.

10. A secondary battery, comprising an electrolyte according to any one of claims 1-9.

11. A battery module, comprising a secondary battery according to claim 10.

12. A battery pack, comprising a battery module according to claim 11.

13. A power consuming device, comprising at least one of the secondary battery according to claim 10, the battery module according to claim 11 or the battery pack according to claim 12.
